(19) **Europäisches Patentamt / European Patent Office / Office européen des brevets**

(11) **EP 2 721 418 B1**

(12) **EUROPEAN PATENT SPECIFICATION**

(45) Date of publication and mention of the grant of the patent:
**26.08.2020 Bulletin 2020/35**

(21) Application number: **12729522.8**

(22) Date of filing: **15.06.2012**

(51) Int Cl.:
*G01N 33/72* (2006.01)     *G01N 33/49* (2006.01)
*A61B 5/1455* (2006.01)

(86) International application number:
**PCT/EP2012/061532**

(87) International publication number:
**WO 2012/172097 (20.12.2012 Gazette 2012/51)**

(54) **METHOD FOR DETERMINING A PLASMA VOLUME VARIATION AND DEVICES FOR IMPLEMENTING THE METHOD**

VERFAHREN ZUR BESTIMMUNG EINER PLASMAVOLUMENABWEICHUNG UND VORRICHTUNG ZUR IMPLEMENTIERUNG DES VERFAHRENS

PROCÉDÉ POUR DÉTERMINER UNE VARIATION DE VOLUME DE PLASMA ET DISPOSITIFS POUR LA MISE EN OEUVRE DU PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2011 EP 11305764**

(43) Date of publication of application:
**23.04.2014 Bulletin 2014/17**

(73) Proprietors:
- **INSERM (Institut National de la Santé et de la Recherche Médicale)**
  **75013 Paris (FR)**
- **Université de Lorraine**
  **54000 Nancy (FR)**
- **Centre Hospitalier Et Universitaire De Nancy (CHU)**
  **54035 Nancy Cedex (FR)**

(72) Inventors:
- **ZANNAD, Faiez**
  **F-54500 Vandoeuvre Les Nancy (FR)**
- **ROSSIGNOL, Patrick**
  **F-54500 Vandoeuvre Les Nancy (FR)**

(74) Representative: **Plasseraud IP**
**66, rue de la Chaussée d'Antin**
**75440 Paris Cedex 09 (FR)**

(56) References cited:

**WO-A2-98/03847**     **US-A1- 2006 252 670**

- **BELIN DE CHANTEMELE E. ET AL.: "Blood volume measurement: The comparison of pulse dye densitometry and Dill and Costill's methods", LIFE SCI., vol. 78, no. 14, 28 February 2006 (2006-02-28), pages 1564-1569, XP028050569,**
- **PAPADEA C. ET AL.: "Evaluation of the i-STAT Portable Clinical Analyzer for point-of-care blood testing in the intensive care units of a university children's hospital", ANN. CLIN. LAB. SCI., vol. 32, no. 3, 2002, pages 231-243, XP008090972,**
- **Anonymous: "Hemopoint H2 : Accurate hemoglobin and hematocrit results with one simple test.", , 2009, XP002664180, Retrieved from the Internet: URL:http://www.stanbio.com/media/pdf/attac hments/hemopoint_h2_sell_sheet.pdf [retrieved on 2011-11-18]**
- **NIELSEN F.T. ET AL.: "Inhibition of mineralocorticoid receptors with eplerenone alleviates short-term cyclosporin A nephrotoxicity in conscious rats", NEPHROL. DIALYS. TRANSPLANT., vol. 23, no. 9, September 2008 (2008-09), pages 2777-2783, XP002664181,**
- **ROSSIGNOL P. ET AL.: "Eplerenone survival benefits in heart failure patients post-myocardial infarction are independent from its diuretic and potassium-sparing effects", J. AM. COLLEGE CARDIOL., vol. 58, no. 19, 1 November 2011 (2011-11-01), pages 1958-1966, XP055012577,**

EP 2 721 418 B1

**Description**

The present invention relates to a method for determining a plasma volume variation in humans, and applications of the same.

Although recommended by current congestive heart failure (HF) management guidelines, long term diuretic use is still a matter of controversy, since it may be associated with worse prognosis and is not supported by large-scale randomized controlled studies. However, congestion is associated with worse outcome in patients with HF.

In outpatients, congestion is routinely evaluated with physical examination (jugular vein pressure, weight gain, edema, rales) which are imprecise and have poor sensitivity, or BNP or NT pro-BNP (B-type natriuretic peptide) measurements which are highly variable on a day-to-day basis and have specificity problems, influenced, also by kidney function (being defined as the glomerular filtration rate) and age.

A study named "Eplerenone Post-Acute Myocardial Infarction Heart Failure Efficacy and Survival Study" (EPHESUS) evaluated the effects of eplerenone (a mineralocorticoid receptor blocker diuretic compound) on morbidity and mortality among patients with acute myocardial infarction (MI) complicated by left ventricular dysfunction and heart failure.

The EPHESUS study underlined that the addition of a low-dose of eplerenone to standard medical therapy (including other diuretics) in patients with acute myocardial infarction and heart failure with left ventricular systolic dysfunction improved survival by 15%, with significant reductions in cardiovascular death, sudden death and hospitalization for heart failure. In addition to a variety of pleiotropic effects, eplerenone exerts a diuretic, as well as a potassium-sparing effect.

The inventors have considered the results of this study and they have notably focused attention to know whether a diuretic effect (defined as a weight and a plasma volume variation) and/or a potassium-sparing (K-sparing) effect could be detected in patients treated with eplerenone in an EPHESUS substudy and, if any, whether these effects influenced cardiovascular outcomes.

The inventors have found that independently from eplerenone use, and without any significant interaction, estimated plasma volume depletion was consistently significantly associated with a 11-19 % improvement in most of the tested cardiovascular outcomes (all-cause death, cardiovascular death or cardiovascular hospitalization, all-cause death or cardiovascular hospitalization, hospitalization for heart failure) but not to sudden death. Accordingly, they discovered that the assessment of the plasma volume variation is a good parameter for monitoring the progression of heart failure in a patient.

It would therefore be particularly desirable to provide a method for determining a plasma volume variation in order to monitor the progression of heart failure in a patient treated for this condition or not.

A subject of the present application is therefore a method for evaluating the risk of a patient suffering from heart failure of having a cardiovascular event, by determining in vitro the plasma volume variation $\Delta V$ between time t2 and time t1, t2 being different from time t1, then by comparing the plasma volume variation $\Delta V$ to a reference value $\Delta V_{threshold}$ selected from 1.4 to 20%, wherein a plasma volume variation exceeding the reference value $\Delta V_{threshold}$, is associated with a risk of having a cardiovascular event.

"Human parameters" are parameters used for determining the value of another parameter named "physiological human parameter". Human parameters are for example: Weight, Size; parameters obtained from blood such as Total red blood cells, Hemoglobin, Hematocrit or packed cell volume (PCV), Mean corpuscular volume (MCV) of the red cells, Total white blood cells, Mean platelet volume (MPV) etc.

Suitable fluid samples are for example urine, plasma, serum or whole blood.

Preferably, one or both or all of the two or more parameters is measured on the same type of a human physiological fluid sample, preferably from a whole blood sample.

The two or more human parameters may be selected from the group comprising hemoglobin level, hematocrit level, weight of a patient, etc. For example, hematocrit and weight or hematocrit and hemoglobin levels may be used.

A patient subjected to the above in vitro tests is preferably a patient suffering from congestive heart failure, or hypertension, chronic kidney insufficiency, or cirrhosis.

The variation of the plasma volume $\Delta V$ can be determined according any mathematical formula well-known from a person skilled in the art, from the values obtained.

Preferably, the plasma volume variation $\Delta V$ between time t1 and time t2 is determined using hemoglobin level and hematocrit level as human parameters in combination with the Strauss formula given below (Strauss MB, Davis RK, Rosenbaum JD, Rossmeisl EC. Water diuresis produced during recumbency by the intravenous infusion of isotonic saline solution. J Clin Invest 1951;30:862-8 ; Kalra PR, Anagnostopoulos C, Bolger AP, Coats AJ, Anker SD. The regulation and measurement of plasma volume in heart failure. J Am Coll Cardiol 2002;39:1901-8):

$$\Delta V = 100 \times \frac{hemoglobin\ (t1)}{hemoglobin\ (t2)} \times \frac{1 - hematocrit\ (t2)}{1 - hematocrit\ (t1)} - 100$$

Suitable commercial apparatus allowing to measure hemoglobin level and hematocrit level include for example the apparatus commercialized by Stanbio Laboratory under the trade name HemoPoint® H2 Analyzer, the Chemistry Analyzer commercialized by Roche Diagnostics under the trade name Reflotron® Plus and the test for hematocrit as part of the i-STAT® system of Abbott.

A variation of a plasma volume $\Delta V$ can be also determined from the determination of plasma volumes at different times. A plasma volume can be determined for example from the hematocrit level and weight of a patient, according to Kaplan's formula given below:

$$PV= 0.065x \text{ weight } x(1-hematocrit)$$

 For example, PVt2-PVt1 allows obtaining $\Delta V$.

Therefore, under other conditions for the implementation of the invention, the method for determining in vitro a physiological human parameter in a patient comprises the steps consisting of:

i) determining a plasma volume from a human physiological fluid sample and weight of said patient at time t1,
ii) determining a plasma volume from a physiological fluid sample and weight of said patient at time t2 different from time t1, and
iii) determining a plasma volume variation $\Delta V$ between time t2 and time t1.

Preferably, the two or more human parameters allow determining plasma volumes ($PV_{t1}$, $PV_{t2}$) at times t1 and t2.

The delay between t2 and t1 cannot be nil. In other words, time t2 is necessarily different from time t1 and reasonably spaced from time t1 such that a possible plasma volume variation $\Delta V$ can be noticed.

The delay between time t2 and time t1 may vary according to a condition whose evolution is to be assessed. The delay may also vary upon numerous factors such as the severity of the treated disease, the age and relative health of the subject, the sex of the subject, the potency of the compound used in a treatment such as a diuretic treatment, the indication towards which the treatment is directed. Suitable delays are for example every day, every two days, every week, every two weeks or every month or more.

The delay between t2 and t1 is preferably between one day and two months, particularly between two days and two months, more particularly between one week and one month.

It should be noted that in the present application, in a standard fashion the indefinite article "a" must be considered as a generic plural (meaning "at least one" or also "one or more"), except when the context indicates the contrary (1 or "one only"). Thus, for example, when it says above that the method comprises the determination of a plasma volume, this refers to the measure of one or more plasma volumes for example in order to check the consistency of the plasma volume measured value.

The reference value (of $\Delta V$) can be defined as a threshold being a percentage of $\Delta V$. It can also be defined as an absolute value of $\Delta V$. The reference value of $\Delta V_{threshold}$ is between 1.4 and 20%, preferably between 13 and 17%, more preferably about 15%.

Taking into the account the variation coefficient of the device (the point of care) of 10%, the reference value is preferably about 5%.

Taking into the account the variation of the device (the point of care) of 1%, the reference value is preferably about 1.4%.

Under other preferred conditions for implementation of the invention, the method further comprises the determination of a serum potassium level of a patient and preferably the comparison of said value to a serum potassium reference value.

A usual range considered as normal is between 3,8 and 5,4 mmol/L.

Under other preferred conditions for implementation of the invention, the method further comprises the determination of a serum creatinine level of the patient and preferably the comparison of said value to a creatinine reference value.

A usual range considered as normal for the creatinine level mainly depends from age, gender.

For example, a usual range considered as normal for the serum creatinine level is between 50 and 90pmol/L for a female and between 70 and 120pmol/L for a male.

Creatinine level assessment notably allows one to estimate the glomerular filtration rate, by using for example the MDRD formula (Modification of the Diet in Renal Disease) -Levey 2000- which depends on patient age and gender.

Under other preferred conditions for implementation of the invention, the method further comprises the determination of glomerular filtration rate variation of the patient and preferably the comparison of said value to glomerular filtration rate variation reference value.

The reference value (of glomerular filtration rate variation) can be defined as a threshold being a percentage of glomerular filtration rate variation. It can also be defined as an absolute value of serum creatinine level variation. The reference value of glomerular filtration rate variation threshold is preferably about 20%.

Under other preferred conditions for implementation of the invention, the method further comprises the determination of a serum BNP level or serum NT-ProBNP level of a patient and preferably the comparison of said value to a serum BNP reference value or a serum NT-ProBNP reference value respectively.

For patients presenting with acute onset or worsening of symptoms, the exclusion cutoff is 300 pg/mlfor NT-proBNP and 100 pg/ml for BNP. For patients presenting in a non-acute way, the optimum exclusion cut-off point is 125 pg/mL for NT-proBNP and 35 pg/mL for BNP. Generally, whenever a physiological parameter may be assessed from different physiological samples including whole blood, the latter is preferred.

The present disclosure further describes a device for carrying out the methods of the present invention, particularly methods including two or more tests made on human physiological fluid samples.

Therefore, the present disclosure also describes a device for performing the above method comprising:

- means for determining values of two or more parameters from a human physiological fluid sample at time t1;
- means for determining values of two or more parameters from a human physiological fluid sample at time t2 different from time t1; and
- means for determining a plasma volume variation $\Delta V$ between time t2 and time t1 from the values obtained at times t1 and t2.

The device preferably further comprises means for comparing said plasma volume variation with a reference value, particularly with a reference value $\Delta V_{threshold}$ selected from 1.4 to 20%.

Means for determining two or more parameters such as hematocrit level and hemoglobin level at time t1 and means for determining two or more same parameters at time t2 can be the same means since the determinations are made at different times.

For example hematocrit level and hemoglobin level can be determined by a same apparatus such as Hemo-Point® H2 Analyzer which, using a single drop of blood, offers results for both hemoglobin and hematocrit (calculated) tests in a few seconds. The test for hematocrit as part of the i-STAT® system of Abbott provides hematocrit level and calculated hemoglobin level. The Chemistry Analyzer commercialized by Roche Diagnostics under the trade name Reflotron® Plus allows determination of 17 parameters from whole blood, serum or plasma.

According to another instance, the plasma volume variation can be determined from the plasma volumes measured (according to formulae well-known from the man skilled in the art) at times t1 and t2 (see above).

Therefore, under other conditions the device for performing the method comprises:

- means for determining plasma volume from a human physiological fluid sample at time t1,
- means for determining plasma volume from a human physiological fluid sample at time t2 different from time t1; and
- means for calculating a plasma volume variation.

The means for calculating plasma volume variation are preferably calculators which can be programmed according to the formula selected to calculate the plasma volume variation.

Preferably, the calculators may be programmed according the Strauss formula from the recorded values for both hemoglobin and hematocrit at selected times.

The man skilled in the art of computer softwares knows how to program calculators according the formula chosen to calculate a plasma volume variation or compare a plasma volume variation to a reference value or a value given at a previous timepoint.

According to another instance, the device further comprises means for entering or modifying a reference value including for example a keyboard. A port or a wireless system for example using the Bluetooth® technology may also allow entering or modifying a reference value from a separate computer. The different values and results of calculations are preferably recorded in a memory.

The value of the plasma volume variation can be displayed on the device or can be simply stocked in a memory in the device or both.

According to another preferred instance, the device further comprises means for alerting a user whether the subject is at risk of having a cardiovascular event when the plasma volume variation exceeds the reference value $\Delta V_{threshold}$.

Means for alerting can be visuals or sonorous. These means for alerting a user can also allow sending data to a computer of the medical specialist via internet. Such means can for example be wireless.

Under other preferred conditions the device for performing the method further comprises means for determining serum potassium level of a physiological sample and preferably means for comparing serum potassium level with a reference value.

Under other preferred conditions the device further comprises means for displaying potassium level.

Potassium level can be determined by a same apparatus as the one used for the determination of hemoglobin and hematocrit levels or by a different apparatus. Potassium level can be determined by potentiometry. A kit may include an apparatus for hemoglobin and hematocrit test and an apparatus for potassium test. A suitable test for potassium may be implemented using analytical plates of Vitros Chemistry Products K+.

Under still other preferred conditions the device further comprises means for alerting if the potassium level exceeds a reference value.

Under still other preferred conditions the device for performing the method further comprises:

- means for determining serum creatinine level of a physiological fluid sample; and
- preferably means for comparing serum creatinine level with a reference value or with patient previous value.

Creatinine level can be determined by a same apparatus as the one used for the determination of hemoglobin and hematocrit levels or by a different apparatus such as StatSensor® creatinine meter.

Under other preferred conditions the device further comprises means for displaying creatinine level.

Under other preferred conditions the device further comprises means for alerting a user if the creatinine level exceeds the reference value.

Under still other preferred conditions the device for performing the method further comprises:

- means for determining serum BNP level or serum NT-ProBNP level of a physiological fluid sample; and
- possibly means for comparing serum BNP level or serum NT-ProBNP level with a reference value or with patient previous value.

Serum BNP level or serum NT-ProBNP level can be determined by a same apparatus as the one used for the determination of hemoglobin and hematocrit levels or by a different apparatus such as i-STAT®.

Under other preferred the device further comprises means for displaying serum BNP level or serum NT-ProBNP level.

Under other preferred conditions the device further comprises means for alerting a user if the serum BNP level or serum NT-ProBNP level exceeds the reference value.

The method which is the subject of the present invention possess very useful properties. It makes it possible to provide a reliable assessment of plasma volume variation and optionally or additionally of potassium level, creatinine level or serum BNP level or serum NT-ProBNP level.

These properties justify the use of the methods described above, in a method for monitoring the progression of a condition where plasma volume variation is representative of the condition or of its evolution, such as heart failure in a patient, and more particularly, for determining whether a heart failure post myocardial infarction patient having a treatment (in particular decongestive) is at risk of having a cardiovascular event such as death, cardiovascular death, cardiovascular hospitalization, hospitalization for heart failure.

The methods described above find a use particularly in a method for assessing the results of the treatment of different conditions such as hypertension, chronic kidney insufficiency, or cirrhosis i.e. diseases involving plasma volume variation, preferably involving plasma volume and potassium variations and more preferably involving plasma volume, potassium and kidney function variations.

The methods described above find also a use in a method for assessing the results of the treatment of different non therapeutic conditions involving plasma volume variation.

This is why the subject of the present invention is a method for determining the progression of a condition where plasma volume variation is representative of the condition or its evolution comprising the method steps defined above.

The method is a method for determining whether a patient suffering from heart failure, particularly after post myocardial infarction, is at risk of having a cardiovascular event comprising the method steps defined above.

According to the method, it is particularly possible to know whether a patient is at risk of having a cardiovascular event when the plasma volume variation is higher than said reference value corresponding to a given threshold value.

The reference value which can be a threshold value may be determined by the skilled person according to the patient, for example taking account sex, age, weight...

When the skilled person is aware that the plasma volume variation has exceeded the reference value, a modification in his treatment can be done or a treatment can be instituted.

However, in order to change for example the dosage of drugs, for some conditions such as heart failure, it is interesting if the skilled person has also knowledge of the potassium or creatinine level (or both of course) of the patient. Under other preferred condition, it is interesting if the skilled person has also knowledge of the potassium, creatinine level, BNP level or NT-ProBNP level of the patient.

Therefore, under preferred conditions for the implementation of the invention, the above method for determining the progression of a condition where plasma volume variation is representative of the condition or its evolution further comprises the step consisting of determining the potassium level of said patient and preferably the step consisting of comparing said potassium level to a reference value. This assessment helps particularly when a treatment comprises using K-sparing agents or K- depleting diuretics.

Under other preferred conditions for the implementation of the invention, the above method for determining the progression of a condition where plasma volume variation is representative of the condition or its evolution further comprises the step consisting of determining the creatinine level of said patient and preferably the step consisting of comparing said creatinine level to a reference value.

The assessment of the creatinine level is particularly interesting since it allows the determination of the glomerular filtration rate which itself allows estimating the kidney function being a strong predictor of mortality and morbidity in general populations as well as in heart failure patients. Indeed, Kidney function may influence both serum potassium levels, and drugs efficiency and side effects. It is therefore preferable to take its variations into account when adapting patient treatment.

Under other preferred conditions for the implementation of the invention, the above method for determining the progression of a condition where plasma volume variation is representative of the condition or its evolution further comprises the step consisting of determining the serum BNP level or serum NT-ProBNP level of said patient and preferably the step consisting of comparing said serum BNP level or serum NT-ProBNP level to a reference value.

The present invention is now illustrated with the following examples.

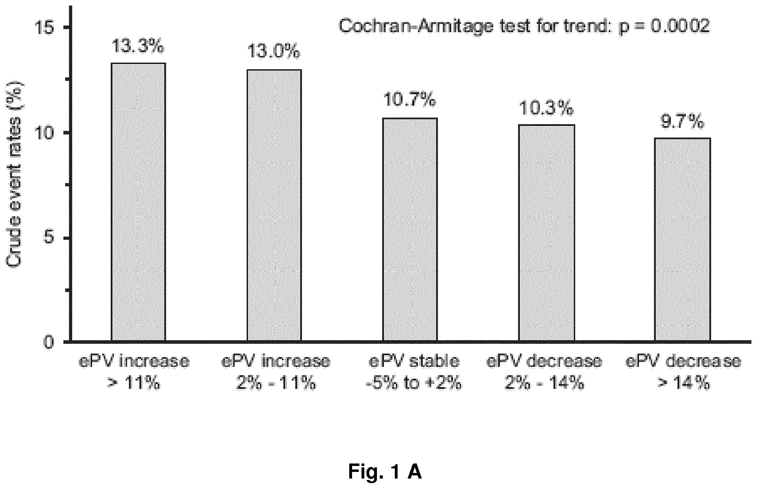

Figure 1A represents all-cause death according to ePV (estimated plasma volume) change from base line.

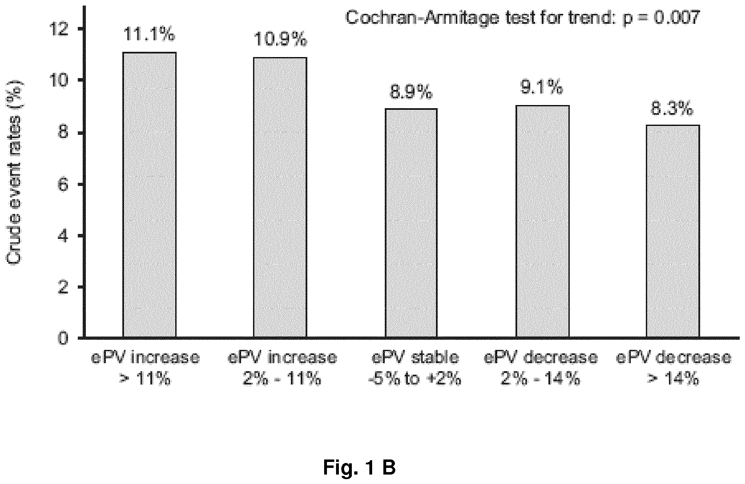

Figure 1B represents cardiovascular death according to ePV (estimated plasma volume) change from base line.

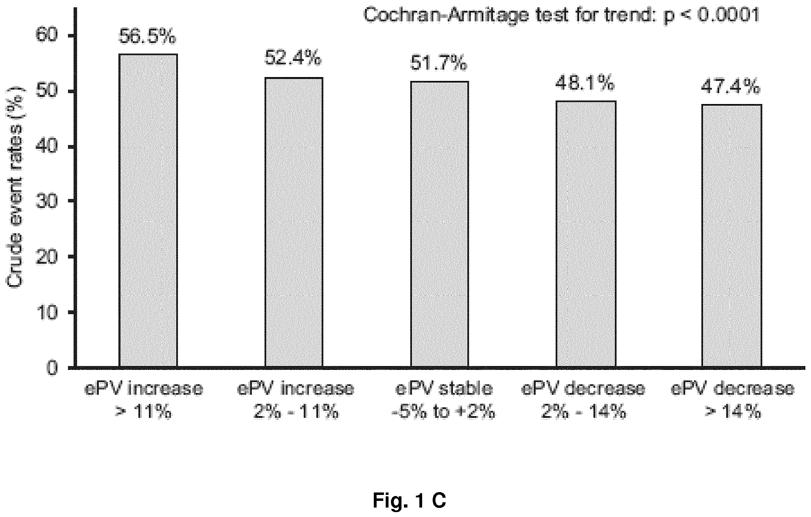

Figure 1C represents all-cause death/hospitalization according to ePV (estimated plasma volume) change from base line.

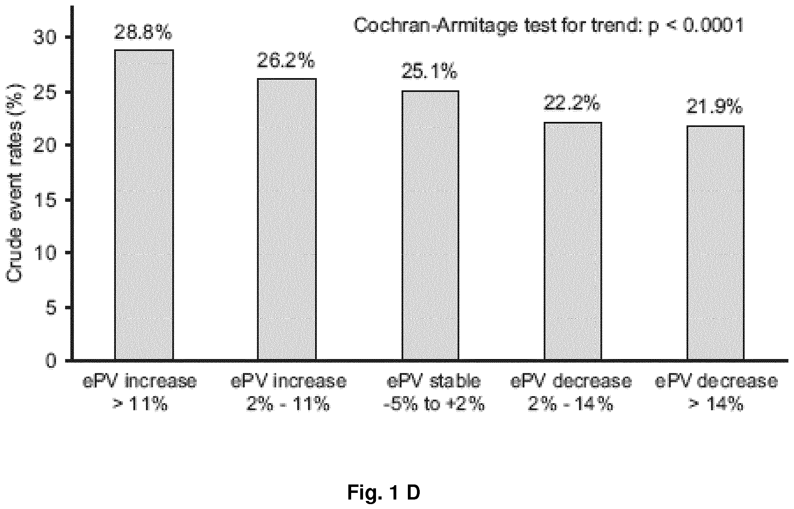

Figure 1D represents cardiovascular death/hospitalization according to ePV (estimated plasma volume) change from base line.

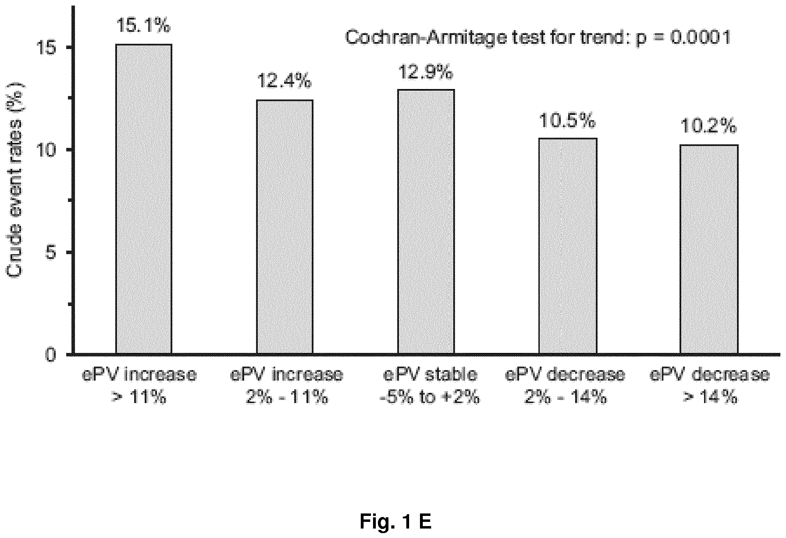

Figure 1E represents hospitalization for unplanned heart failure according to ePV (estimated plasma volume) change from base line.

## Examples

### 1. Association between quintiles of estimated plasma volume changes at month 1 and the cardiovascular outcomes, in a whole study population

As previously mentioned, a study named "Eplerenone Post-Acute Myocardial Infarction Heart Failure Efficacy and Survival Study" (EPHESUS) evaluated the effects of eplerenone (a mineralocorticoid receptor blocker diuretic compound) on morbidity and mortality among patients with acute myocardial infarction (MI) complicated by left ventricular dysfunction and heart failure.

The inventors determined whether a diuretic-effect may be detectable in the mineralocorticoid receptor antagonist eplerenone-treated patients as compared to placebo during the first month of EPHESUS (n= 6080) and whether this was associated with eplerenone's beneficial effects on cardiovascular outcomes.

Figures 1A-1E show that across the spectrum of the tested cardiovascular outcomes (besides sudden death), the association between the intensity of the estimated plasma volume depletion and the crude event rates showed a significant linear trend in the whole study population.

Accordingly, assessment of plasma volume variation is representative of the evolution of the condition and may be targeted for treatment adaptation.

Moreover, the inventors have shown both potassium variations and kidney function variations between inclusion and month 1 were also associated with the cardiovascular outcomes (Rossignol P, Cleland JG, Bhandari S, Tala S, Gustafsson F, Fay R, Lamiral Z, Dobre D, Pitt B, Zannad F. Determinants and consequences of renal function variations with aldosterone blocker therapy in heart failure patients after myocardial infarction: insights from the Eplerenone Post-Acute Myocardial Infarction Heart Failure Efficacy and Survival Study. Circulation. 2012 Jan 17;125(2):271-279). It is therefore also interesting to assess the value of creatinine level and/or potassium level.

### 2. Monitoring of the progression of the disease in patients suffering of a heart failure post myocardial infarction.

Using an HemoPoint® H2 Analyzer, hemoglobin and hematocrit levels were obtained from 12 patients suffering from heart failure and treated by diuretics and inhibitors of the renin-angiotensin-aldosterone system every two weeks.

From hemoglobin and hematocrit levels, plasma volumes and their variations were calculated.

Some patients were tested for serum potassium by potentiometry using analytical plates of Vitros Chemistry Products K+.

The results obtained evidence that 1 month (t2) after initiating (t1) eplerenone plasma volume V dropped by 2 % and K+ rose to 5.9 mmol/L and creatinine rose by 20%.It appeared necessary to decrease the eplerenone dose used in these patients because of hyperkalemia, worsening renal function.

In other patients, an increase in plasma volume of 22% was associated with a creatinine increase of 2% (which however remained in the normal range), an hypokalaemia (potassium 3.5 mmol/L), which allowed to increase eplerenone dose, with subsequent improvement of plasma volume and serum potassium.

## Claims

1. A method for evaluating the risk of a patient suffering from heart failure of having a cardiovascular event, by determining in vitro the plasma volume variation $\Delta V$ between time t2 and time t1, t2 being different from time t1, then by comparing the plasma volume variation $\Delta V$ to a reference value $\Delta V_{threshold}$ selected from 1.4 to 20%, wherein a plasma volume variation exceeding the reference value $\Delta V_{threshold}$, is associated with a risk of having a cardiovascular event.

2. The method according to claim 1 wherein the plasma volume variation $\Delta V$ between time t2 and time t1 is determined from hemoglobin level and/or haematocrit level.

3. The method according to claim 1 wherein the plasma volume variation $\Delta V$ between time t2 and time t1 is determined from hemoglobin level and haematocrit level.

4. The method according to anyone of claims 1-3 further comprising the determination of serum potassium level measured from a physiological fluid sample.

5. The method according to claim 4 further comprising the step of comparing the serum potassium level to a serum potassium reference value.

6. The method according to one of claims 1-5 further comprising the determination of serum creatinine level measured from a physiological fluid sample.

7. The method according to claim 6 further comprising the step of comparing the serum creatinine level to a serum creatinine level reference value.

## Patentansprüche

1. Verfahren zum Bewerten des Risikos eines Patienten, der an Herzinsuffizienz leidet, ein kardiovaskuläres Ereignis zu haben, durch Bestimmen der Plasmavolumenvariation $\Delta V$ zwischen dem Zeitpunkt t2 und dem Zeitpunkt t1 in vitro, wobei t2 von dem Zeitpunkt t1 unterschiedlich ist, dann durch Vergleichen der Plasmavolumenvariation $\Delta V$ mit einem Referenzwert $\Delta V_{Schwellenwert}$, ausgewählt aus 1,4 bis 20%, wobei eine Plasmavolumenvariation, die den Referenzwert $\Delta V_{Schwellenwert}$ übersteigt, mit einem Risiko verbunden ist, ein kardiovaskuläres Ereignis aufzuweisen.

2. Verfahren nach Anspruch 1, wobei die Plasmavolumenvariation $\Delta V$ zwischen dem Zeitpunkt t2 und dem Zeitpunkt t1 aus dem Hämoglobinspiegel und/oder dem Hämatokritspiegel bestimmt wird.

3. Verfahren nach Anspruch 1, wobei die Plasmavolumenvariation $\Delta V$ zwischen dem Zeitpunkt t2 und dem Zeitpunkt t1 aus dem Hämoglobinspiegel und dem Hämatokritspiegel bestimmt wird.

4. Verfahren nach einem der Ansprüche 1 - 3 weiterhin umfassend die Bestimmung des Serumkaliumspiegels, gemessen von einer physiologischen Flüssigkeitsprobe.

5. Verfahren nach Anspruch 4 weiterhin umfassend den Schritt Vergleichen des Serumkaliumspiegels mit einem Serumkalium-Referenzwert.

6. Verfahren nach einem der Ansprüche 1 - 5 weiterhin umfassend die Bestimmung des Serumkreatininspiegels, gemessen von einer physiologischen Flüssigkeitsprobe.

7. Verfahren nach Anspruch 6 weiterhin umfassend den Schritt Vergleichen des Serumkreatininspiegels mit einem Serumkreatininspiegel-Referenzwert.

**Revendications**

1. Procédé d'évaluation du risque pour un patient souffrant d'insuffisance cardiaque d'avoir un accident cardiovasculaire, en déterminant *in vitro* la variation du volume plasmatique $\Delta_v$ entre un temps $t_2$ et un temps $t_1$, $t_2$ étant différent du temps $t_1$, puis en comparant ladite variation du volume plasmatique $\Delta_v$ à une valeur de référence $\Delta_{Vseuil}$ choisie de 1,4 à 20%, dans lequel une variation du volume plasmatique dépassant la valeur de référence $\Delta_{Vseuil}$, est associée à un risque d'avoir un accident cardiovasculaire.

2. Procédé selon la revendication 1 dans lequel la variation du volume plasmatique $\Delta_v$ entre le temps $t_2$ et le temps $t_1$ est déterminée à partir du taux d'hémoglobine et/ou du taux d'hématocrite.

3. Procédé selon la revendication 1 dans lequel la variation du volume plasmatique $\Delta_v$ entre le temps $t_2$ et le temps $t_1$ est déterminée à partir du taux d'hémoglobine et du taux d'hématocrite.

4. Procédé selon l'une des revendications 1 à 3 comprenant en outre la détermination du taux de potassium sérique mesuré à partir d'un échantillon de fluide physiologique.

5. Procédé selon la revendication 4 comprenant en outre une étape de comparaison du taux de potassium sérique à une valeur de référence du potassium sérique.

6. Procédé selon l'une des revendications 1 à 5 comprenant en outre la détermination du taux de créatinine sérique mesuré à partir d'un échantillon de fluide physiologique.

7. Procédé selon la revendication 6 comprenant en outre une étape de comparaison du taux de sérum créatinine sérique à une valeur de référence de la créatinine sérique.

**Fig. 1 A**

Fig. 1 B

Fig. 1 C

**Fig. 1 D**

EP 2 721 418 B1

Fig. 1 E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **STRAUSS MB ; DAVIS RK ; ROSENBAUM JD ; ROSSMEISL EC.** Water diuresis produced during recumbency by the intravenous infusion of isotonic saline solution. *J Clin Invest,* 1951, vol. 30, 862-8

- **KALRA PR ; ANAGNOSTOPOULOS C ; BOLGER AP ; COATS AJ ; ANKER SD.** The regulation and measurement of plasma volume in heart failure. *J Am Coll Cardiol,* 2002, vol. 39, 1901-8
- the Eplerenone Post-Acute Myocardial Infarction Heart Failure Efficacy and Survival Study. *Circulation,* 17 January 2012, vol. 125 (2), 271-279